# EUROPÄISCHE PATENTANMELDUNG

(11) **EP 0 988 827 A1**
(43) Veröffentlichungstag der Anmeldung: **29.03.2000**
(21) Anmeldenummer: 99118145.4
(22) Anmeldetag: 11.09.1999
(51) Int. Cl.: A61B 5/0428, A61B 5/0416

(54) **Anschlussvorrichtung für die intraatriale EKG-Ableitung**

(30) Priorität: 23.09.1998 DE 29817053 U
(71) Anmelder: B. BRAUN MELSUNGEN AG, 34212 Melsungen (DE)
(72) Erfinder: Seibert, Richard L., Allentown Pennsylvania 18103 (US)
(74) Vertreter: Selting, Günther, Dipl.-Ing.

(57) **Zusammenfassung**

Die Anschlußvorrichtung weist ein Patientenkabel (11) auf, mit dem EKG-Klammern (18,19,20) an EKG-Haftelektroden (21) angeschlossen werden können, um eine extrakorporale EKG-Ableitung durchzuführen. Für eine intraatriale EKG-Ableitung ist ein Adapter (34) vorgesehen, der mit einer EKG-Klammer (39) ausgestattet ist und an den eine EKG-Klammer (19) des Patientenkabels (11) angeschlossen werden kann. Der Adapter weist einen zweiten Eingang (40) auf, an den ein Anschlußkabel (26) angeschlossen werden kann. Der zugehörige Konnektor (28) des Anschlußkabels (26) ist so ausgebildet, daß er wahlweise direkt mit einer EKG-Klammer (19) konnektiert werden kann, ohne daß der Adapter (34) vorhanden ist.

## Beschreibung

Die Erfindung betrifft eine Anschlußvorrichtung für die intraatriale EKG-Ableitung und insbesondere eine Anschlußvorrichtung, mit der das Patientenkabel eines EKG-Monitors mit dem elektrischen Leiter eines Cava-Katheters verbunden werden kann.

Eine Anschlußvorrichtung, von der der Oberbegriff des Anspruchs 1 ausgeht, ist bekannt aus EP 0 596 344 B1. Die bekannte Anschlußvorrichtung besteht aus einem Anschlußkabel, das mit dem elektrischen Leiter eines Cava-Katheters unmittelbar verbunden werden kann und dadurch in Kontakt mit dem Atrium gelangt, und einem Adapter, an den ein Konnektor des Patientenkabels ansteckbar ist und der mit einer EKG-Klammer des zu einem Monitor gehörenden Patientenkabels verbindbar ist. Bei ausschließlicher Verwendung des Patientenkabels kann eine extrakorporale EKG-Ableitung durchgeführt werden, indem die drei EKG-Klammern des Patientenkabels an EKG-Elektroden angesteckt werden, die auf dem Körper des Patienten befestigt worden sind. Durch die zusätzliche Verwendung des Adapters besteht die Möglichkeit, anstelle der extrakorporalen EKG-Ableitung oder zusätzlich zu dieser eine intraatriale EKG-Ableitung durchzuführen, bei der ein elektrischer Leiter, der in einem Cava-Katheter verläuft, bis in das Atrium des Patienten vorgeschoben wird. Das extrakorporale Ende des elektrischen Leiters wird über das Anschlußkabel mit einem zweiten Eingang des Adapters verbunden. Es besteht die Möglichkeit, entweder den ersten oder den zweiten Eingang des Adapters auf den Ausgang des Adapters durchzuschalten und das betreffende Potential über die angeschlossene EKG-Klammer des Patientenkabels an den Monitor zu leiten. Die Durchführung der intraatrialen EKG-Ableitung setzt das Vorhandensein des Adapters zwingend voraus. Ohne den Adapter ist das Anschlußkabel nutzlos, weil dann mit Hilfe des Patientenkabels nur ein extrakorporales EKG aufgenommen werden kann.

Katheter-Führungsdrähte, die dazu geeignet sind, mit ihrer aus einem Cava-Katheter vorstehenden Spitze bis zum Herzen vorgeschoben zu werden, sind beschrieben in EP 0 486 979 B1 und in DE 43 19 033 C1. Ferner ist in DE 43 18 963 C1 eine Vorrichtung zur EKG-Ableitung beschrieben, bei der zur Übertragung der EKG-Signale eine Elektrolytflüssigkeit benutzt wird, die eine elektrisch leitende Flüssigkeitssäule bildet.

In EP 0 786 266 A1 ist ein Katheterbesteck mit EKG-Ableitungsmöglichkeit beschrieben, bei welchem die EKG-Signale über einen Führungsdraht aus dem Körper herausgeführt werden. An den Führungsdraht wird seitlich eine mit einem Anschlußkabel verbundene Klammer angesetzt.

Der vorliegenden Erfindung liegt die Aufgabe zugrunde, eine aus Anschlußkabel und Adapter bestehende Anschlußvorrichtung zu schaffen, die wahlweise mit oder ohne Adapter zur Ableitung eines intraatrialen EKG benutzt werden kann.

Die Lösung dieser Aufgabe erfolgt erfindungsgemäß mit den im Anspruch 1 angegebenen Merkmalen.

Bei der erfindungsgemäßen Anschlußvorrichtung ist der zweite Konnektor des Anschlußkabels ein Dual-Konnektor, der zwei Anschlußmöglichkeiten vorsieht. Dieser Konnektor ist nämlich mit einem Aufsteckteil versehen, welcher mit dem Steckteil des Adapters zusammenpaßt, und ferner mit einem leitenden Schaftteil, an den die EKG-Klammer passend anklemmbar ist. Ein wesentliccher Vorteil besteht darin, daß der zweite Konnektor wahlweise mit dem Adapter oder mit einer EKG-Klammer des Patientenkabels verbunden werden kann. Dabei wird davon ausgegangen, daß die EKG-Klammer des Adapters im wesentlichen gleich ausgebildet ist wie die EKG-Klammern des Patientenkabels oder zumindest die gleiche Klemmweite hat. Daher können an dem Schaftteil des zweiten Konnektors des Anschlußkabels entweder die EKG-Klammer des Patientenkabels oder diejenige des Adaptere angreifen. Das Patientenkabel bietet also verschiedene Möglichkeiten der Übertragung intraatrial abgeleiteter EKG-Signale an das Patientenkabel. Die Hauptmöglichkeit besteht natürlich darin, den Konnektor mit seinem Aufsteckteil an dem Steckteil des Adapters zu befestigen. Andererseits besteht auch die Möglichkeit, das Patientenkabel unmittelbar mit dem zweiten Konnektor des Anschlußkabels zu verbinden.

Die Erfindung betrifft ferner eine Anschlußvorrichtung für die intraatriale EKG-Ableitung, welche aus einem normalen Patientenkabel mit mehreren EKG-Klammern und dem erfindungsgemäßen Anschlußkabel besteht. In diesem Fall ist ein Adapter nicht vorhanden, kann jedoch wahlweise eingesetzt werden.

Im folgenden werden Ausführungsbeis unter Bezugnahme auf die Zeichnungen näher erläutert.

Es zeigen:
- Fig. 1: verschiedene Möglichkeiten der EKG-Ableitung über ein Patientenkabel mit Hilfe eines Adapters mit und ohne Anschlußkabel,
- Fig. 2: eine Seitenansicht des zweiten Konnektors des Anschlußkabels, teilweise geschnitten, und
- Fig. 3: eine andere Ausführungsform des Anschlußkabels mit einer auf einen Katheter-Führungsdraht seitlich aufsteckbaren Klemme.

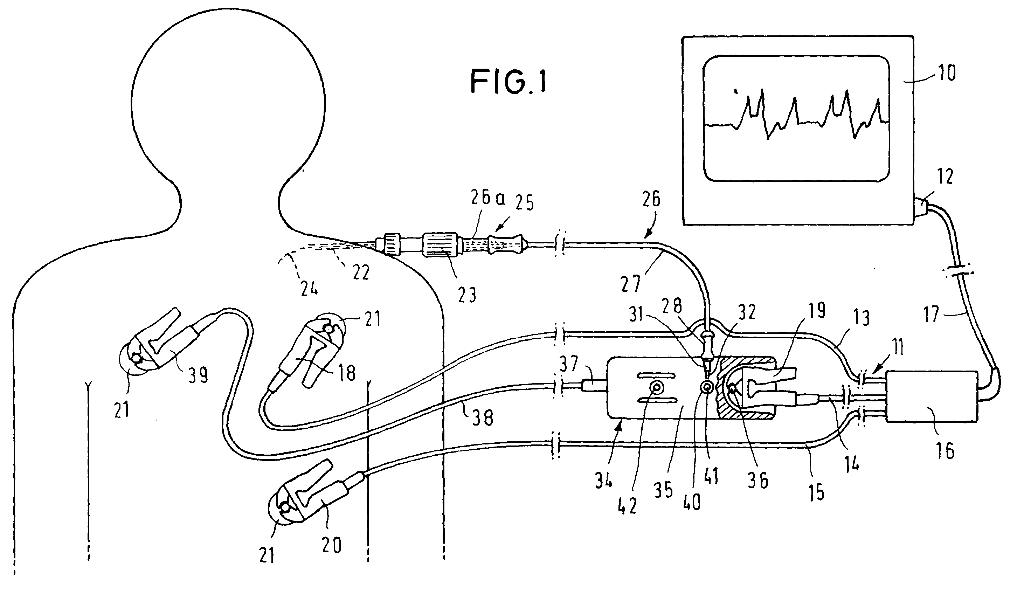

In Fig. 1 ist ein EKG-Monitor 10 dargestellt, mit dem EKG-Spannungen sichtbar gemacht bzw. aufgezeichnet werden können. An den Eingang des EKG-Monitors 10 ist das Patientenkabel 11 mit einem Vielfachstecker 12 angeschlossen. Das Patientenkabel 11 weist bei dem vorliegenden Ausführungsbeispiel drei Leitungen 13,14,15 auf, die mit einem Verteiler 16 verbunden sind, von dem eine mehradrige Leitung 17 zu dem Vielfachstecker 12 führt. Jede der Leitungen 13,14,15 weist an ihrem Ende eine EKG-Klammer 18,19,20 auf, die nach Art einer Zange mit zwei federnd gegeneinandergedrückten Klammerbeinen ausgebildet ist. Üblicherweise wird jede EKG-Klammer 18,19,20 an eine EKG-Elektrode 21 angeklemmt, die auf dem Körper des Patienten befestigt wird.

Ein Cava-Katheter 22 zur intraatrialen EKG-Ableitung ist mit einem Anschlußstück 23 versehen. Durch den Cava-Katheter 22 hindurch verläuft ein Führungsdraht 24, an dem das Anschlußstück 23 fixiert ist. Das Anschlußstück 23 ist generell in gleicher Weise ausgebildet wie dies in EP 0 486 979 B1 beschrieben ist. Das Anschlußstück 23 enthält einen mit dem Führungsdraht 24 verbundenen elektrischen Leiter mit einem Stift, auf den eine metallische Hülse eines Konnektors 25 aufgeschoben werden kann. Diese Hülse ist mit einem isolierenden Mantel 26a umgeben, der zugleich einen Berührungsschutz darstellt.

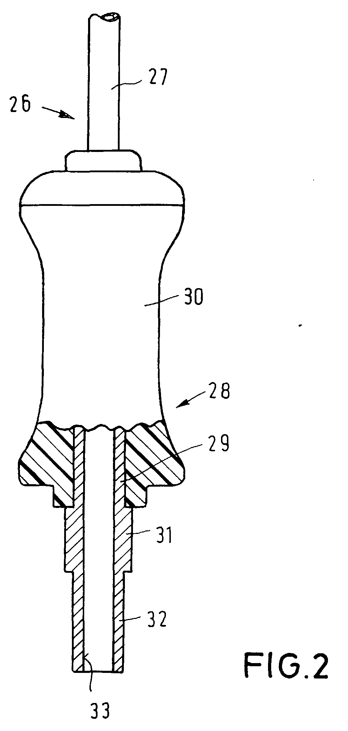

Der Konnektor 25 ist Bestandteil eines Anschlußkabels 26, welches eine isolierte einadrige Leitung 27 aufweist. An dem anderen Ende des Anschlußkabels 26 befindet sich der zweite Konnektor 28, der in Fig. 2 dargestellt ist. Der zweite Konnektor 28 weist eine langgestreckte elektrisch leitende Hülse 29 auf, welche in einen Griffteil 30 aus Kunststoff hineinragt und dort mit der Ader der Leitung 27 verbunden ist. In unmittelbarem Anschluß an den Griffteil 30 ist die Hülse 29 mit einem Schaftteil 31 versehen, der einen vergrößerten Außendurchmesser hat. Koaxial an den Schaftteil 31 schließt sich ein Aufsteckteil 32 von verringertem Außendurchmesser an. Der Außendurchmesser des Schaftteils 31 beträgt 3,4 mm und derjenige des Aufsteckteils 32 beträgt 2,5 mm. Durch die Hülse 29 erstreckt sich eine Bohrung 33 mit einem Durchmesser von 1,5 mm. Die Länge des Aufsteckteils 32 ist etwas größer als diejenige des Schaftteils 31.

Gemäß Fig. 1 ist ein Adapter 34 vorgesehen, an den die mit der Leitung 14 verbundene EKG-Klammer 19 des Patientenkabels 11 angeschlossen werden kann. Der Adapter 34 weist ein aus Kunststoff bestehendes Gehäuse 35 auf, an dem ein elektrischer Ausgang 36 vorgesehen ist, welcher die Form eines Stiftes hat, an den die EKG-Klammer 19 angesetzt werden kann. Der Adapter kann noch mehrere Ausgänge für unterschiedliche Arten von EKG-Klammern haben und somit einen Universaladapter nach EP 0 596 344 B1 bilden.

Der Adapter 35 ist mit einem ersten Eingang 37 versehen, an den über ein Kabel 38 eine EKG-Klammer 39 angeschlossen ist. Auch diese EKG-Klammer 39 ist dazu bestimmt, an eine EKG-Elektrode 21 angesetzt zu werden. Der Adapter 34 weist ferner einen zweiten Eingang 40 auf, welcher aus einem hinter einer Öffnung des Gehäuses angeordneten stiftförmigen Steckteil 41 besteht. Mit einem Schalter 42 kann wahlweise der erste Eingang 37 oder der zweite Eingang 40 auf den Ausgang 36 durchgeschaltet werden. Alternativ besteht die Möglichkeit, den ersten Eingang und/oder den zweiten Eingang permanent mit dem Ausgang 36 zu verbinden.

Der Konnektor 28 kann mit seinem Aufsteckteil 32 auf den Steckteil 41 des Adapters aufgesteckt und mit diesem elektrisch verbunden werden. Dabei wird der Aufsteckteil durch die Gehäuseöffnung des Adapters hindurchgesteckt. Vorzugsweise ist die Gehäuseöffnung so groß, daß auch der Schaftteil 31 hindurchpaßt, so daß der Griffteil 30 aus Isoliermaterial unmittelbar auf dem Gehäuse 35 aufsitzt.

Bei Benutzung der Anschlußvorrichtung werden die EKG-Elektroden 21 am Körper des Patienten befestigt. Dann werden die EKG-Klammern 18 und 20 des Patientenkabels 11 an die betreffenden Haftelektroden angeschlossen. Die EKG-Klammer 19 des Patientenkabels, die für die Ableitung des Brustwand-EKG bestimmt ist, wird in die passende Fassung des Adapters 34 eingesteckt und anstelle dieser EKG-Klammer wird die EKG-Klammer 39 mit der EKG-Elektrode 21 für das Brustwand-EKG verbunden. Schließlich wird das Anschlußkabel 26 sowohl mit dem Katheteranschluß 23 als auch mit dem Adapter 34 verbunden. Durch Betätigung des Umschalters 42 kann ausgewählt werden, ob der Eingang 37 oder der Eingang 40 auf den Ausgang 36 zum Monitor 10 durchgeschaltet wird. Die Leitungen 13,15 sind über den Verteiler 16 ständig mit dem EKG-Monitor 10 verbunden.

Wenn das Anschlußkabel 26 ohne den Adapter 34 benutzt werden soll, wird die EKG-Klammer 19 des Patientenkabels mit dem zweiten Konnektor 28 verbunden. Dies geschieht dadurch, daß die Zangenbeine der EKG-Klammer 19 an den Schaftteil 31 angesetzt werden und diesen klemmend festhalten. Der Außendurchmesser des Schaftteils 31 ist ausreichend groß, um von der EKG-Klammer 19 festgehalten zu werden.

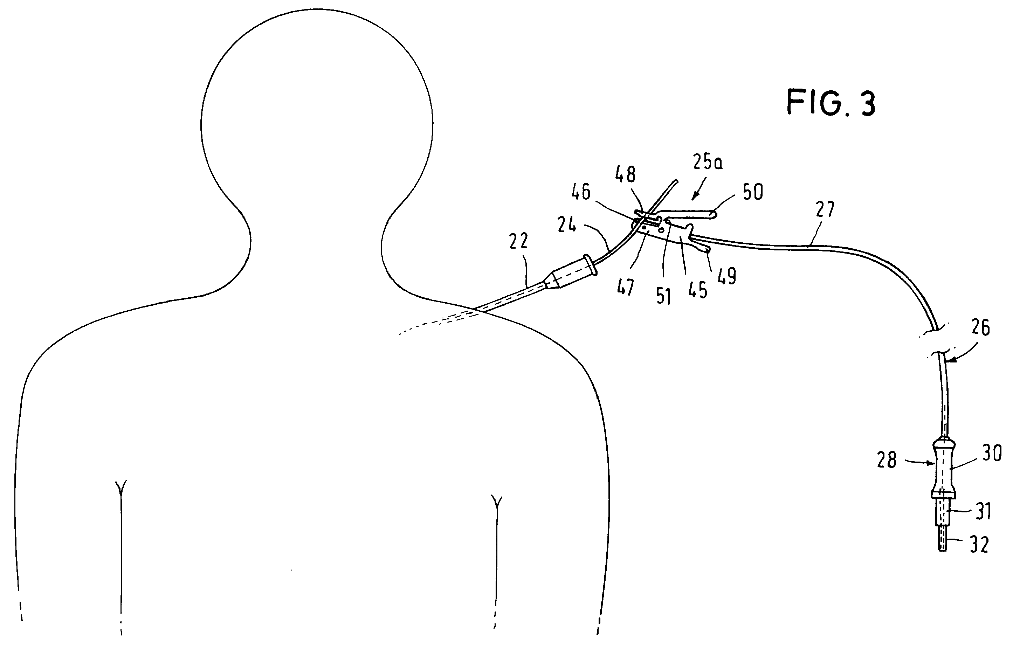

In Fig. 3 ist eine andere Ausführungsform des Anschlußkabels 26 dargestellt. Der zweite Konnektor 28 ist in gleicher Weise ausgebildet wie derjenige des ersten Ausführungsbeispiels, jedoch unterscheidet sich der erste Konnektor 25a von dem Konnektor 25. Der Konnektor 25a ist eine Klemme, die seitlich auf den aus dem Cava-Katheter 22 herausragenden Abschnitt des Führungsdrahtes 24 aufgesteckt wird und die elektrischen EKG-Signale von diesem Führungsdraht 24 abnimmt. Die Klemme 45 weist eine Metallplatte 46 auf, die von einem Halteschenkel 47 abgestützt ist. Ein Andrückschenkel 48 drückt den Führungsdraht 24 gegen die Metallplatte 46, die mit der Seele der Leitung 27 verbunden ist. Auf diese Weise wird das elektrische Potential an dem Führungsdraht 24 abgegriffen und über das Anschlußkabel 26 dem EKG-Monitor 10 zugeführt. Die beiden Schenkel 47,48 sind Bestandteil der aus Kunststoff bestehenden Klemme 45, die zwei Griffschenkel 49,50 aufweist, welche durch ein Filmscharnier 51 mit den Schenkeln 47,48 verbunden sind. Durch Zusammendrücken der Griffschenkel 49,50 werden die Schenkel 47,48 auseinanderbewegt, um den Führungsdraht 24 zu umgreifen. Werden die Griffschenkel anschließend losgelassen, wird der Führungsdraht an der Metallplatte 46 eingeklemmt. Die Klemme 25a kann an jeder Position des freiliegenden Abschnitts des Führrungsdrahtes 24 angeklemmt werden.

## Patentansprüche

1. Anschlußvorrichtung für die intraatriale EKG-Ableitung, mit einem Anschlußkabel (26), das an einem Ende einen ersten Konnektor (25;25a) für den Anschluß an den elektrischen Leiter eines Cava-Katheters (22) und am anderen Ende einen zweiten Konnektor (28) aufweist, und mit einem Adapter (34), der einen mit einer EKG-Klammer (39) verbundenen ersten Eingang (37), einen mit dem zweiten Konnektor (28) des Anschlußkabels (26) verbindbaren zweiten Eingang (40) und einen mit dem Patientenkabel (11) eines Monitors (10) verbindbaren Ausgang (36) aufweist,
**dadurch gekennzeichnet,**
daß der zweite Konnektor (28) des Anschlußkabels (26) einen mit einem Steckteil (41) des Adapters (34) zusammenpassenden Aufsteckteil (32) und einen leitenden Schaftteil (31) zum Anklemmen an einer EKG-Klammer (19) aufweist.

2. Anschlußvorrichtung für die intraatriale EKG-Ableitung, mit einem Anschlußkabel (26), das an einem Ende einen ersten Konnektor (25;25a) für den Anschluß an den elektrischen Leiter eines Cava-Katheters (22) und am anderen Ende einen zweiten Konnektor (28) aufweist, und mit einem Patientenkabel (11), das mindestens eine EKG-Klammer (19) mit einem EKG-Monitor (10) verbindet, dadurch gekennzeichnet, daß der zweite Konnektor (28) des Anschlußkabels (26) einen mit einem Steckteil (41) des Adapters (34) zusammenpassenden Aufsteckteil (32) und einen leitenden Schaftteil (31), an den die EKG-Klammer (19) des Patientenkabels (11) passend anklemmbar ist, aufweist.

3. Anschlußvorrichtung nach Anspruch 1 oder 2, dadurch gekennzeichnet, daß der erste Konnektor (25a) des Anschlußkabels (26) eine auf einen Katheter-Führungsdraht (24) seitlich aufsteckbare Klemme (45) ist.

4. Anschlußvorrichtung nach Anspruch 1 oder 2, dadurch gekennzeichnet, daß der erste Konnektor (25) des Anschlußkabels (26) eine nach außen isolierte Kontakthülse aufweist.

5. Anschlußvorrichtung nach einem der Ansprüche 1-4, dadurch gekennzeichnet, daß der Schaftteil (31) und der Aufsteckteil (32) koaxial zueinander in gegenseitiger Verlängerung angeordnet sind.

6. Anschlußvorrichtung nach einem der Ansprüche 1-5, dadurch gekennzeichnet, daß der Schaftteil (31) einen größeren Außendurchmesser hat als der Aufsteckteil (32).

7. Anschlußvorrichtung nach einem der Ansprüche 1-6, dadurch gekennzeichnet, daß der Aufsteckteil (32) als Hülse ausgebildet ist, die klemmend über ein stiftförmiges Steckteil (41) des Adapters (34) schiebbar ist.
